(19) Europäisches Patentamt
European Patent Office
Office européen des brevets

(11) Publication number: **0 017 680**
**B1**

(12) # EUROPEAN PATENT SPECIFICATION

(45) Date of publication of patent specification: **21.04.82**

(21) Application number: **79300600.8**

(22) Date of filing: **11.04.79**

(51) Int. Cl.³: **C 07 D 401/14,**
C 07 D 417/14,
A 61 K 31/505

(54) **Pyrimidone derivatives, process for preparing them and pharmaceutical compositions containing them.**

(43) Date of publication of application:
**29.10.80 Bulletin 80/22**

(45) Publication of the grant of the patent:
**21.04.82 Bulletin 82/16**

(84) Designated Contracting States:
**BE CH DE FR GB IT LU NL SE**

(56) References cited:
**FR - A - 2 336 935**

(73) Proprietor: **SMITH KLINE & FRENCH LABORATORIES LIMITED**
**Mundells**
**Welwyn Garden City Hertfordshire, AL7 1EY (GB)**

(72) Inventor: **Brown, Thomas Henry**
**115 Daniells**
**Welwyn Garden City Hertfordshire (GB)**
Inventor: **Durant, Graham John**
**401 Knightsfield**
**Welwyn Garden City Hertfordshire (GB)**
Inventor: **Ganellin, Charon Robin**
**"Kinwood" Briary Wood End**
**Welwyn Hertfordshire (GB)**
Inventor: **Ife, Robert John**
**19 Mobbsbury Way**
**Stevenage Hertfordshire (GB)**

(74) Representative: **Waters, David Martin, Dr. et al,**
**P.O. Box 39**
**Welwyn Garden City, Hertfordshire AL7 1EY (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 017 680 B1

# Pyrimidone derivatives, process for preparing them and pharmaceutical compositions containing them

This invention relates to pharmacologically active pyrimidone derivatives, a process for preparing them, and pharmaceutical compositions containing them.

According to the present invention there is provided a pyrimidone of Structure (1)

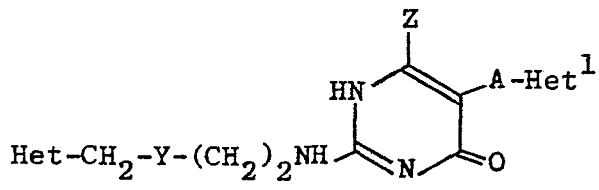

(1)

in which Het is a 2- or 4- imidazolyl group optionally substituted by lower alkyl (preferably methyl), halogen (preferably chlorine or bromine), trifluoromethyl or hydroxymethyl, a 2-pyridyl group optionally substituted by one or more (which can be the same or different) lower alkyl (preferably methyl), lower alkoxy (preferably methoxy), halogen (preferably chlorine or bromine), amino or hydroxy groups, a 2-thiazolyl group, a 3-isothiazolyl group optionally substituted by chlorine or bromine, a 3-(1,2,5)-thiadiazolyl group optionally substituted by chlorine or bromine, or a 2-(5-amino-1,3,4-thiadiazolyl) group; Y is sulphur or methylene; Z is hydrogen or lower alkyl (preferably methyl); A is $C_1$—$C_5$ alkylene; and Het[1] is a pyridyl group substituted by hydroxy or N-oxo and optionally substituted by lower alkyl, or lower alkoxy.

Throughout this specification by the terms 'lower alkyl' and 'lower alkoxy' is meant alkyl and alkoxy groups containing 1 to 4 carbon atoms which can be straight or branched. Particular lower alkyl groups are methyl, ethyl, 1-propyl and 2-propyl. Particular lower alkoxy groups are methoxy, ethoxy, 1-propoxy and 2-propoxy.

Preferably Het is a 5-methyl-4-imidazolyl, 5-bromo-4-imidazolyl, 2-pyridyl, 3-methyl-2-pyridyl, 3-methoxy-2-pyridyl, 3-ethoxy-2-pyridyl, 3,4-dimethoxy-2-pyridyl, 3-fluoro-2-pyridyl, 3-chloro-2-pyridyl, 3-bromo-2-pyridyl, 3-iodo-2-pyridyl, 3-bromo-4-methyl-2-pyridyl or 2-thiazolyl group.

Preferably Z is hydrogen.

Preferably A is straight $\alpha,\omega$-alkylene, particularly methylene.

Particular meanings of Het[1] are 4-hydroxy-2-pyridyl, 6-hydroxy-3-pyridyl, 2-hydroxy-4-pyridyl, 4-hydroxy-5-methyl-2-pyridyl, 6-hydroxy-5-methyl-3-pyridyl, 2-hydroxy-6-methyl-4-pyridyl, 6-hydroxy-5-methoxy-3-pyridyl, N-oxo-3-pyridyl, N-oxo-6-methyl-3-pyridyl, and N-oxo-4-pyridyl.

Preferably Het[1] is a pyridyl group substituted by hydroxy and optionally substituted by lower alkyl, and particularly 4-hydroxy-2-pyridyl, 6-hydroxy-3-pyridyl or 2-hydroxy-4-pyridyl.

The compounds of the invention are shown and described as 4-pyrimidones and these exist in equilibrium with the corresponding 6-pyrimidones, and to a lesser extent in the following tautomeric forms:

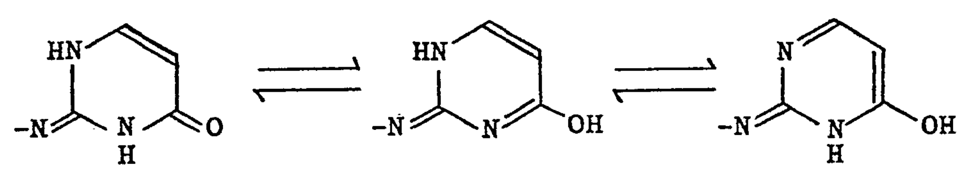

The 2, 4- and 6-hydroxypyridyl groups can exist as 1H-pyrimidone tautomers, and certain of the Het groups can exist in several tautomeric forms. All these tautomeric forms of the compounds of Structure 1 are within the scope of the present invention.

In a process of the invention a compound of Structure (1) is prepared by reacting a compound of Structure (3);

$$Het\text{-}CH_2\text{-}Y\text{-}(CH_2)_2NH_2$$

(2)

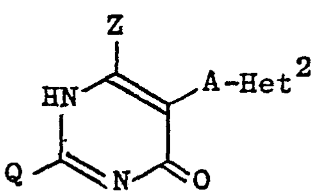

(3)

in which Q is nitroamino ($NO_2NH$—), lower alkylthio, benzylthio, chlorine, bromine or other group which can be displaced with a primary amine, Z and A are as defined for Structure (1), and Het[2] is a pyridyl

group substituted by hydroxy, a protected hydroxy group or N-oxo and optionally substituted by lower alkyl or lower alkoxy, with an amine of Structure (2), in which Het and Y are as defined for Structure (1) and removal of any hydroxy protecting groups.

This reaction can be carried out at an elevated temperature in the absence of a solvent, for example at 80°C to 170°C, preferably 120°C to 140°C, or in a solvent at an elevated temperature, for example at the reflux temperature of the reaction mixture. The choice of solvent is affected by solubility characteristics of the reactants and the particular meaning of Q. Preferably the solvent is pyridine, a picoline or mixture of picolines, a lower alkanol, preferably ethanol or 1-propanol, an aqueous mixture of a lower alkanol, 1,2-ethanediol, a ketone, for example acetone or 2-butanone, or a polar aprotic solvent for example dimethylformamide, dimethylacetamide, dimethylsulphoxide, hexamethylphosphoramide, sulpholane, acetonitrile or nitromethane. Particularly preferably Q is nitroamino and the reaction is carried out in refluxing ethanol, refluxing 1-propanol or refluxing pyridine, or Q is methylthio and the reaction is carried out in refluxing pyridine.

Preferably approximately equimolar amounts of the reactants are used, although an excess for example a slight excess of from 1.1 to 1.5 molar equivalents or a larger excess of from 1.5 to 4 molar equivalents, of either reactant can be used. If an excess of a reactant is used then preferably an excess of the amine of Structure (2) is used. An excess of either reactant can be present at the start of the reaction or can be added during the course of the reaction.

Examples of hydroxy protecting groups are methoxymethyl, methylthiomethyl, tetrahydropyranyl, arylmethyl, for example benzyl, lower alkyl, for example methyl, and acyl, for example formyl or acetyl. Compounds of Structure (1) in which $Het^1$ is a 2-, 4- or 6-hydroxypyridyl group can be conveniently prepared from a compound of Structure (3) in which $Het^2$ is a pyridyl group with a 2, 4- or 6-lower alkoxy substituted by acid hydroylsis of the product of the reaction with an amine of Structure (2).

The compounds of Structure (3) in which Q is nitroamino can be prepared by reacting a $\beta$-oxo-ester of Structure (4)

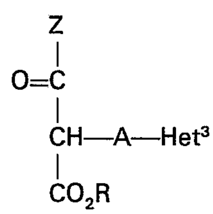

(4)

in which Z is hydrogen or lower alkyl, R is lower alkyl, A is as defined for Structure (1), and $Het^3$ is a pyridyl group optionally substituted by lower alkyl, or lower alkoxy, or a pyridyl group substituted by hydroxy, a protected hydroxy group, or N-oxo and optionally substituted by lower alkyl or lower alkoxy with nitroguanidine, and when $Het^3$ is a pyridyl group optionally substituted by lower alkyl or lower alkoxy, reacting the product with an oxidising agent to give a N-oxo-pyridyl derivative, and optionally removing any hydroxy protecting group present.

The compounds of Structure (3) in which Q is lower alkylthio or benzylthio can be prepared by reacting a $\beta$-oxoester of Structure (4) in which $Het^3$ is a pyridyl group substituted by hydroxy, a protected hydroxy group or N-oxo, and optionally substituted by lower alkyl or lower alkoxy, with thiourea, and alkylating or benzylating the 2-thiouracil formed, and optionally removing any hydroxy protecting group present.

The compounds of Structure (3) in which Q is chlorine or bromine can be prepared by reacting a $\beta$-oxoester of Structure (4) with guanidine and diazotising the product in hydrochloric acid in the presence of cuprous chloride or in hydrobromic acid in the presence of cuprous bromide, and when $Het^3$ is a pyridyl group optionally substituted by lower alkyl or lower alkoxy, reacting the product with an oxidising agent to give a N-oxypyridyl derivative, and optionally removing any hydroxy protecting group present.

Preferably the reactions of the $\beta$-oxoester of Structure (4) with nitroguanidine, thoiurea and guanidine are carried out in the presence of a base, for example, an alkali metal lower alkoxide, preferably sodium methoxide, or sodium ethoxide, an alkali metal carbonate or hydroxide, preferably potassium carbonate or sodium hydroxide, sodium hydride or a quaternary ammonium hydroxide, for example benzyltrimethylammonium hydroxide. Preferably this reaction is carried out at an elevated temperature, for example the reflux temperature of the solvent mixture. Preferably the solvent is a lower alkanol, for example ethanol, an aqueous mixture of a lower alkanol, a ketone, for example 2-butanone, or a polar aprotic solvent, for example dimethylformamide. When Z is hydrogen the $\beta$-oxoester of Structure (4) can be used in the form of a hemiacetal of a lower alkanol.

Preferably a peroxycarboxylic acid, for example 3-chloroperoxybenzoic acid, peroxybenzoic acid or peracetic acid is used as the oxidising agent to convert a pyridyl group into a N-oxo-pyridyl group. Preferably this oxidation is carried out in acetic acid.

The amines of Structure (2) in which Y is sulphur can be prepared by reacting cysteamine with a compound of formula Het-$CH_2L$ where L is a group displaceable with a thiol, for example hydroxy, acyloxy (for example acetoxy, methane-sulphonyloxy or p-toluenesulphonyloxy), lower alkoxy (for example methoxy), chlorine, bromine or trialylphosphonium (for example triphenylphosphonium). Preferably L is hydroxy or methoxy and the reaction is carried out under acidic conditions, for example in hydrochloric or hydrobromic acid.

The amines of Structure (2) in which Y is methylene and Het is a 2-pyridyl group with a lower alkoxy group or halogen atom in the 3-position can be prepared by reacting a 2-halo-3-nitropyridine with diethyl 2-(2-cyanoethyl)-malonate. Hydrolysis and decarboxylation of the product followed by reduction with palladium and charcoal gives a 3-amino-2-(3-cyanopropyl)pyridine which can be diazotised in 2M sulphuric acid and alkylated in dimethyl sulphoxide to give a 3-alkoxy-2-(3-cyanopropyl)pyridine. The 3-amino-2-(3-cyanopropyl)pyridines can be reduced with lithium aluminium hydride to give a 4-(3-amino-2-pyridyl)-butylamine which can be diazotised in strong hydrochloric acid in the presence of cuprous chloride to give a 3-chloro amine, or diazotised in strong hydrobromic acid in the presence of cuprous bromide to give a 3-bromo amine, or diazotised in dilute sulphuric acid containing sodium iodide to give a 3-iodo amine. The 3-amino-2-(3-cyanopropyl)pyridines can be diazotised in fluoroboric acid and reduced with lithium aluminium hydride to give a 4-(3-fluoro-2-pyridyl)butylamine.

The amines of Structure (2) in which Y is methylene and Het is a 2-thiazolyl group can be prepared by reacting a thioamide of structure $NH_2CS(CH_2)_4Q$ where Q is a protected amino group with a dialkyl acetal of bromoacetaldehyde and removal of the amino-protecting group.

The esters of Structure (4) can be prepared by alkylating a dialkyl malonate followed by hydrolysis and decarboxylation, or by condensing an aldehyde with malonic acid and decarboxylating, esterifying and reducing the product.

The compounds of Structure (1) have histamine $H_2$-antagonist activity and also have histamine $H_1$-antagonist activity and are particularly active as histamine $H_2$-antagonists when compared to analogous compounds, for example those described in FR—A—2366935, in which $Het^1$ is other than a pyridyl ring substituted by hydroxy or N-oxo. The compounds of Structure (1) also have a low lipophilicity as measured by octanol-water distribution.

In this specification by histamine $H_2$-receptors is meant receptors defined by Black et al. (Nature, *236*, 385 (1972)) as those histamine receptors which are not blocked by mepyramine but are blocked by burimamide, and by histamine $H_1$-receptors is meant receptors involved in mepyramine-sensitive histamine responses. Compounds which block histamine $H_2$-receptors are referred to as histamine $H_2$antagonists and compounds which block histamine $H_1$-receptors are referred to as histamine $H_1$-antagonists.

Blockade of histamine $H_2$-receptors is of value in inhibiting the biological actions of histamine which are not inhibited by histamine $H_1$-antagonists. Histamine $H_2$-antagonists are active, for example, as inhibitors of gastric acid secretion, an antiinaflammatory agents and as agents which act on the cardiovascular system, for example as inhibitors of the effects of histamine on blood pressure.

In some physiological conditions the biological actions of histamine are mediated through both histamine $H_1$- and $H_2$-receptors and blockage of both types of receptors is useful. These conditions include inflammation mediated by histamine, for example skin inflammation, and those hypersensitivity responses due to the action of histamine at $H_1$- and $H_2$-receptors, for example allergies.

The activity of the compounds of Structure (1) as histamine $H_2$-antagonists can be demonstrated by the inhibition of histamine-stimulated secretion of gastric acid from the lumen-perfused stomachs of rats anaesthetised with urethane, at doses of less than 16 micromoles per kilogram intravenously. This procedure is referred to in Ash and Schild, Brit. J. Pharmac. Chemother, *27*, 427 (1966). Their activity as histamine $H_2$-antagonists can also be demonstrated by their ability to inhibit other actions of histamine which, according to the above mentioned paper of Ash and Schild, are not mediated by histamine $H_1$-receptors. For example, they inhibit the actions of histamine on the isolated guinea pig atrium and isolated rat uterus. They inhibit the basal secretion of gastric acid and also that stimulated by pentagastrin or by food. In a conventional test such as the measurement of blood pressure in the anaesthetised cat, at doses of from 0.5 to 256 micromoles per kilogram intravenously they inhibit the vasodilator action of histamine. The potency of these compounds is illustrated by the effective doses producing 50% inhibition of gastric acid secretion in the anaesthetised rat and the dose producing 50% inhibition of the histamine-induced tachycardia in the isolated guinea pig atrium (less than $10^{-4}$ Molar).

The activity of the compounds of Structure (1) as histamine $H_1$-antagonists can be demonstrated by the inhibition of histamine-stimulated contractions of the isolated guinea-pig ileum. It is advantageous to administer a single compound having histamine $H_1$- and $H_2$-antagonist activity rather than to administer individual compounds having histamine $H_1$-antagonist activity and histamine $H_2$-antagonist activity as difficulties arising from differing rates of absorption and pharmacokinetic characteristics are avoided.

The pharmaceutical compositions of the invention comprise a pharmaceutical carrier and a pharmacologically active compound of Structure (1) which can be in the base form or in the form of an addition salt with a pharmaceutically-acceptable acid. Such addition salts include those with

hydrochloric, hydrobromic, hydriodic, sulphuric and maleic acids and may conveniently be formed from the corresponding compounds of Structure (1) by standard procedures, for example by treating them with an acid in a lower alkanol or by the use of ion exchange resins to form the required salt either directly from the compound in the base form or from a different addition salt.

The pharmaceutical carrier employed can be a solid or liquid. Examples of solid carriers are lactose, maize starch, potato starch, or modified starches, dicalcium phosphate, terra alba, sucrose, celluloses, talc, gelatin, agar, pectin, acacia, magnesium stearate and stearic acid. Examples of liquid carriers are syrup, peanut oil, olive oil, alcohol, propylene glycol, polyethylene glycols and water.

If a solid carrier is used, the composition can be prepared in the form of a tablet, capsule containing powder or pellets, troche or lozenge. The amount of solid carrier in a unit dosage form is generally from about 25 mg to about 300 mg. If a liquid carrier is used, the composition can be in the form of a syrup, emulsion, multiple emulsion, sterile injectable liquid or an aqueous or non-aqueous solution or liquid suspension. Other additives such as preservatives, for example antioxidants or antibacterials, and/or flavouring or colouring agents can also be included. The sterile liquids can be prepared in ampoules, multidose vials or unit dose disposable systems. The preparation can also be in a semi-solid form, for example a cream, paste, ointment or gel, or in a liquid or aerosol form for topical application. The pharmaceutical compositions are prepared by conventional techniques involving procedures such as milling, mixing, granulating and compressing, spray drying, freeze drying or dissolving or dispersing the ingredients as appropriate to the desired preparation. The active ingredient is present in the compositions in an effective amount to block histamine $H_2$-receptors. Preferably, each dosage unit contains the active ingredient in an amount of from about 50 mg to about 250 mg.

The active ingredient is preferably administered one to six times per day. The daily dosage regimen is preferably from about 150 mg to about 1500 mg. The route of administration can be oral or parenteral.

The invention is illustrated by the following Examples in which temperatures are in °C.

Example 1

(a) A mixture of 2-methoxy-5-cyanopyridine (61.26 g), semi-carbazide hydrochloride (76.4 g), sodium acetate (74.92 g), ethanol (1300 ml) and water (400 ml) was hydrogenated at 344 kPa using Raney nickel catalyst (1.0 g). The mixture was evaporated to a volume of 500 ml, water (1000 ml) was added and the mixture was allowed to stand at 0° overnight. The mixture was filtered and the solid was washed with water and dissolved in 10% hydrochloric acid (1000 ml). Formaldehyde solution (36% w/v, 450 ml) was added and the mixture was warmed for 15 minutes, allowed to cool and was added to a solution of sodium acetate (298.5 g) in water (900 ml). This mixture was extracted with ether and the combined extracts were successively washed with aqueous potassium carbonate and water and were dried and evaporated to give 6-methoxypyridine-3-carboxyaldehyde (31.5 g, 50%), m.p. 48—49°.

(b) A mixture of 6-methoxypyridine-3-carboxaldehyde (2.34 g), monoethyl malonate (4.51 g), pyridine (12 ml) and piperidine (6 drops) was heated under reflux for 5 hours and was evaporated to an oil. This oil was partitioned between ether and dilute aqueous ammonia. The ether layer was washed with water and evaporated to an oil which crystallised on standing to give ethyl 3-(6-methoxy-3-pyridyl)acrylate (2.8 g, 79%), m.p. 49—52°.

(c) Ethyl 3-(6-methoxy-3-pyridyl)acrylate (32.33 g) in ethanol (160 ml) was hydrogenated at 344 kPa and 40° using palladium-on-charcoal catalyst (5%, 0.2 g). The mixture was filtered and the filtrate was evaporated to give ethyl 3-(6-methoxy-3-pyridyl)propionate (32.7 g) as an oil.

(d) A mixture of ethyl 3-(6-methoxy-3-pyridyl)propionate (32.74 g) and ethyl formate (17.22 g) was added dropwise over 1.5 hours to a stirred suspension of sodium hydride in oil (50%, 9.38 g) in 1,2-dimethoxyethane (50 ml) cooled to —2°, and allowed to stand overnight at room temperature. The mixture was poured on to ice and the mixture was extracted with ether (discarded), and the aqueous phase was adjusted to pH 5 with 2N sulphuric acid. An oil was precipitated and crystallised on standing to give ethyl 2-formyl-3-(6-methoxy-3-pyridyl)propionate (25.9 g, 70%), m.p. 91.5—94°. A sample recrystallised from aqueous ethanol had m.p. 93—94°C.

(e) Nitroguanidine (4.7 g) was added to a solution of sodium methoxide (prepared from 1.15 g sodium) in methanol (50 ml) and the mixture was boiled under reflux for 45 minutes. Ethyl 2-formyl-3-(6-methoxy-3-pyridyl)propionate (10.7 g) was added and the mixture was refluxed for 34 hours and evaporated to a residue. This residue was dissolved in water and the solution was extracted with chloroform (subsequently discarded). The aqueous solution was adjusted to pH 5 with acetic acid, and the solid which precipitated out was filtered off to give 2-nitroamino-5-(6-methoxy-3-pyridylmethyl)-4-pyrimidone, m.p. 183.5—186°.

(f) An equimolar mixture of 2-nitroamino-5-(6-methoxy-3-pyridylmethyl)-4-pyrimidone and 2-(5-methyl-4-imidazolyl methylthio)-ethylamine was refluxed in ethanol for 18 hours. The solid which crystallised out on cooling was recrystallised from ethanol to give 2-[2-(5-methyl-4-imidazolyl methylthio)ethylamino]-5-(6-methoxy-3-pyridylmethyl)-4-pyrimidone, m.p. 197—198.5° in 63% yield.

(g) 2-[2-(5-Methyl-4-imidazolylmethylthio)ethylamino]-5-(6-methoxy-3-pyridylmethyl)-4-pyrimidone (0.55 g) in 2N hydrogen chloride in ethanol was boiled under reflux for 24 hours. The mixture

was evaporated to dryness and the residue was recrystallised from 2-propanol/ethanol containing hydrogen chloride to give 2-[2-(5-methyl-4-imidazolylmethylthio)ethylamino]-5-(6-hydroxy-3-pyridylmethyl)-4-pyrimidone trihydrochloride, m.p. 205—209° in 71% yield.

Example 2

(a) An equimolar mixture of 2-nitroamino-5-(6-methoxy-3-pyridylmethyl)-4-pyrimidone and 2-(2-thiazolylmethylthio)ethylamine was refluxed in ethanol for 18 hours. The solid which crystallised out on cooling was recrystallised from ethanol to give 2-[2-(2-thiazolylmethylthio)ethylamino]-5-(6-methoxy-3-pyridylmethyl)-4-pyrimidone, m.p. 95—97° in 60% yield.

(b) 2-[2-(2-Thiazolylmethylthio)ethylamino]-5-(6-methoxy-3-pyridylmethyl)-4-pyrimidone in 2N hydrogen chloride in ethanol was boiled under reflux for 24 hours. The mixture was evaporated to dryness and the residue was recrystallised from 2-propanol/ethanol containing hydrogen chloride to give 2-[2-(2-thiazolylmethylthio)ethylamino]-5-(6-hydroxy-3-pyridylmethyl)-4-pyrimidone trihydrochloride, m.p. 200—204°.

Example 3

(a) Sodium (20.8 g) was dissolved in methanol (285 ml), a solution of 2-chloro-4-cyanopyridine (115.53 g) in methanoldioxan (1:1, 850 ml) was added and the mixture was boiled under reflux for $2\frac{1}{2}$ hours and was allowed to cool. The mixture was filtered and the volume of the filtrate was reduced by evaporation to 200 ml and water (400 ml) was added. The solid which precipitated out was filtered off to give 2-methoxy-4-cyanopyridine (57.2 g, 51%), m.p. 93—95.5°.

(b) A mixture of 2-methoxy-4-cyanopyridine (57.2 g), semicarbazide hydrochloride (71.24 g), sodium acetate (69.86 g), ethanol (1200 ml) and water (370 ml) was hydrogenated at 344 kPa using Raney nickel catalyst (1.0 g). The mixture was evaporated to a volume of 450 ml, water (900 ml) was added and the mixture was allowed to stand at 0° overnight. The mixture was filtered and the solid was washed with water and was dissolved in 10% hydrochloric acid (950 ml). Formaldehyde solution (36% w/v, 420 ml) was added and the mixture was warmed for 30 minutes, allowed to cool and was added to a solution of sodium acetate (280 g) in water (840 ml). The mixture was extracted with ether (3 x 500 ml) and the combined extracts were successively washed with aqueous potassium carbonate and water and were dried and evaporated to give 2-methoxypyridine-4-carboxyaldehyde (20.53 g, 35%) m.p. 33—5°. A sample recrystallised from petroleum ether had m.p. 33—36°.

(c) Substitution of 2-methoxypyridine-4-carboxaldehyde for 6-methoxypyridine-3-carboxyaldehyde in the general procedure of Example 1 (b, c, d) gave ethyl 2-formyl-3-(2-methoxy-4-pyridyl)propionate as an oil, and treatment of this with nitroguanidine and sodium methoxide according to the procedure of Example 1(e) gave 2-nitroamino-5-(2-methoxy-4-pyridylmethyl)-4-pyrimidone in 59% yield, m.p. 194—195.5° (from aqueous acetic acid).

(d) An equimolar mixture of 2-nitroamino-5-(2-methoxy-4-pyridylmethyl)-4-pyrimidone and 2-(5-methyl-4-imidazolylmethylthio)ethylamine was heated under reflux in ethanol for 18 hours. The solid which crystallised out on cooling was recrystallised from methanol to give 2-[2-(5-methyl-4-imidazolylmethylthio)ethylamino]-5-(2-methoxy-4-pyridylmethyl)-4-pyrimidone, m.p. 177—178° in 51% yield. The latter compound was heated under reflux in 2N hydrogen chloride in ethanol for 24 hours and the mixture was evaporated to dryness. The residue was recrystallised from 2-propanol/ethanol containing hydrogen chloride to give 2-[2-(5-methyl-4-imidazolylmethylthio)ethylamino]-5-(2-hydroxy-4-pyridylmethyl)-4-pyrimidone trihydrochloride, m.p. 172—176°.

Example 4

An equimolar mixture of 2-nitroamino-5-(2-methoxy-4-pyridylmethyl)-4-pyrimidone and 2-(2-thiazolylmethylthio)ethylamine was heated under reflux in ethanol for 18 hours. The solid which crystallised out on cooling was purified by column chromatography on silica gel and recystallisation from 2-propanol-ethanol to give 2-[2-(2-thiazolylmethylthio)ethylamino]-5-(2-methoxy-4-pyridylmethyl)-4-pyrimidone, m.p. 105.5—106.5° in 41% yield. The latter compound was heated under reflux in 2N hydrogen chloride in ethanol for 24 hours to give 2-[2-(2-thiazolylmethylthio)ethylamino]-5-(2-hydroxy-4-pyridylmethyl)-4-pyrimidone monohydrochloride, m.p. 169—173°C.

Example 5

Reaction of 2-(3-bromo-2-pyridylmethylthio)ethylamine with 1.15 molar equivalents of 2-nitroamino-5-(2-methoxy-4-pyridylmethyl)-4-pyrimidone in refluxing ethanol for 18 hours gave 2-[2-(3 - bromo - 2 - pyridylmethylthio)ethylamino] - 5 - (2 - methoxy - 4 - pyridylmethyl) - 4 - pyrimidone m.p. 70—72° which was boiled under reflux in ethanol containing hydrogen chloride to give 2-[2-(3-bromo-2-pyridylmethylthio)ethylamino]-5-(2-hydroxy-4-pyridylmethyl)-4-pyrimidone trihydrochloride, m.p. 195—198.5°.

Example 6

(a) Substitution of 4 - methoxypyridine - 2 - carboxaldehyde for 6 - methoxypyridine - 3 - carboxaldehyde in the procedure of Example 1(b)(c) gave ethyl 3-(4-methoxy-2-pyridyl)propionate as

an oil which was formylated with ethyl formate and sodium hydride in 1,2-dimethoxyethane and reacted with nitroguanidine and sodium ethoxide to give 2-nitroamino-5-(4-methoxy-2-pyridylmethyl)-4-pyrimidone, m.p. 196—198° (decomp) (from ethanol-acetic acid).

(b) Reaction of 2-nitroamino-5-(4-methoxy-2-pyridylmethyl)-4-pyrimidone with 1.06 molar equivalents of 2-(5-methyl-4-imidazolylmethylthio)ethylamine in refluxing ethanol for 24 hours gave 2-[2-(5-methyl-4-imidazolylmethylthio)ethylamino]-5-(4-methoxy-2-pyridylmethyl)-4-pyrimidone, m.p. 128—130° (from 2-propanol).

(c) A mixture of 2-[2-(5-methyl-4-imidazolylmethylthio)ethylamino]-5-(4-methoxy-2-pyridylmethyl)-4-pyrimidone (0.97 g) and aqueous hydrobromic acid (48%, 20 ml) was boiled under reflux for 20 hours and evaporated to dryness. The residue was recrystallised from a mixture of ethanol and 2-propanol to give 2-[2-(5-methyl-4-imidazolylmethylthio)ethylamino]-5-(4-hydroxy-2-pyridylmethyl)-4-pyrimidone trihydrobromide, m.p. 167—169°. On recrystallisation from a mixture of ethanol and 2-propanol the melting point of the sample was lower owing to loss of hydrogen bromide.

## Examples 7 and 8

(a) Sodium (1.15 g) was dissolved in methanol (50 ml) and nitroguanidine (4.7 g) was added to the cooled solution. The mixture was heated under reflux for 45 minutes, ethyl 2-formyl-3-(3-pyridyl)propionate (9.3 g) was added portionwise and the mixture was heated under reflux for 45 hours and evaporated to dryness. Water was added to the residue and the mixture was extracted with chloroform. The residual aqueous phase was adjusted to pH 5 with acetic acid, and the solid which was precipitated was filtered off, washed and dried to give 2-nitroamino-5-(3-pyridylmethyl)-4-pyrimidone, m.p. 214.5—216° in 38% yield.

(b) 3-Chloroperoxybenzoic acid (10.35 g) was added to 2-nitroamino-5-(3-pyridylmethyl)-4-pyrimidone (12.35 g) in acetic acid (300 ml) and the mixture was stirred at room temperature for 18 hours and at 60° for 5 hours and allowed to cool to room temperature. The solid was filtered off and purified by precipitation from solution in dilute sodium hydroxide by the addition of hydrochloric acid to give 2-nitroamino-5-(N-oxo-3-pyridylmethyl)-4-pyrimidone, m.p. 271° (decomp).

(c) Reaction of 2-nitroamino-5-(N-oxo-3-pyridylmethyl)-4-pyrimidone with one equivalent of

(i) 2-(5-methyl-4-imidazolylmethylthio)ethylamine and

(ii) 4-(chloro-2-pyridyl)butylamine, in refluxing ethanol gave

7. 2-[2-(5-methyl-4-imidazolylmethylthio)ethylamino]-5-(N-oxo-3-pyridylmethyl)-4-pyrimidone, m.p. 95—97°, and
8. 2-[4-(3-chloro-2-pyridyl)butylamino]-5-(N-oxo-3-pyridylmethyl)-4-pyrimidone, m.p. 84—86°.

## Example 9

(a) A mixture of 6-methylpyridine-3-carboxyaldehyde (51.57 g), malonic acid (44.30 g), piperadine (6 ml) and pyridine (300 ml) was stirred at 100° for 3 hours and was allowed to cool. The mixture was evaporated to dryness, water was added to the residue and the solid was filtered off and recrystallised from ethanol-acetic acid to give 3-(6-methyl-4-pyridyl)acrylic acid (41.25 g), m.p. 213.5—215.5°.

(b) A stirred mixture of 3-(6-methyl-3-pyridyl)acrylic acid (50.70 g) dry ethanol (350 ml) and concentrated sulphuric acid (25 ml) was heated under reflux for 18 hours and ethanol (~250 ml) was removed by evaporation. The residue was poured into ice-aqueous ammonia and the mixture was extracted with ether. The ether extracts were washed with water and evaporated to an oil which crystallised on standing to give ethyl 3-(6-methyl-3-pyridyl)acrylate, m.p. 36—37°.

(c) Ethyl 3-(6-methyl-3-pyridyl)acrylate (60.36 g) was hydrogenated in ethanol at 35°C and 355 kPa using palladium-on charcoal catalyst (10%, 1.0 g). The mixture was filtered and the filtrate was evaporated to give ethyl 3-(6-methyl-3-pyridyl)propionate as an oil.

(d) A mixture of ethyl 3-(6-methyl-3-pyridyl)propionate (1.31 g) and ethyl formate (7.43 g) was added dropwise to a stirred suspension of sodium hydride (50% dispersion in oil, 4.07 g) in dry 1,2-dimethoxyethane (24 ml) maintained at 0°. The mixture was allowed to warm to room temperature, stirred overnight and poured into ice-water (300 g). The mixture was extracted with ether, the aqueous phase was adjusted to pH 5.4 with hydrochloric acid, and the solid which separated was collected to give ethyl 2-formyl-3-(6-methyl-3-pyridyl)propionate (10.5 g, 70%), m.p. 142—4°.

(e) A solution of ethyl 2-formyl-3-(6-methyl-3-pyridyl)-propionate (1.55 g) in methanol (20 ml) was added to a stirred solution of sodium methoxide (from 0.161 g sodium) in methanol (20 ml). Dried nitroguanidine (0.73 g) was added and the mixture was heated under reflux overnight and evaporated to dryness. The residue was dissolved in water (50 ml) and the solution was extracted with chloroform and the aqueous phase was adjusted to pH 5 with acetic acid. The solid which precipitated was filtered off and recrystallised from methanol-acetic acid to give 2-nitroamino-5-(6-methyl-3-pyridylmethyl)-4-pyrimidone (0.5 g, 27%) m.p. 215—6° (decomp).

(f) Reaction of 2 - nitroamino - 5 - (6 - methyl - 3 - pyridylmethyl) - 4 - pyrimidone with 3 - chloroperoxybenzoic acid in acetic acid gave 2-nitroamino-5-(N-oxo-6-methyl-3-pyridylmethyl)-4-pyrimidone, m.p. 232° (decomp).

(g) Reaction of 2-nitroamino-5-(N-oxo-6-methyl-3-pyridylmethyl)-4-pyrimidone with one equivalent of 4-(3-chloro-2-pyridyl)butylamine in refluxing ethanol gives 2-[4-(3-chloro-2-pyridyl)butylamino]-5-(N-oxo-6-methyl-3-pyridylmethyl)-4-pyrimidone.

Example 10

Substitution of 4 - (3 - methoxy - 3 - pyridyl)butylamine for 2 - (5 - methyl - 4 - imidazolylmethylthio)ethylamine in the procedure of Example 3(d) gave 2 - [4 - (3 - methoxy - 2 - pyridyl)-butylamino] - 5 - (2 - methoxy - 4 - pyridylmethyl) - 4 - pyrimidone, m.p. 72—74° (from aqueous 2-propanol) which was heated under reflux in 2N hydrogen chloride in ethanl to give 2-[4-(3-methoxy-2-pyridyl)butylamino]-5-(2-hydroxy-4-pyridylmethyl)-4-pyrimidine trihydrochloride m.p. 168—171°.

Examples 11 to 13

Substitution of (i) 4-(2-pyridyl)butylamine
(ii) 4-(3-chloro-2-pyridyl)butylamine
(iii) 4-(3-bromo-2-pyridyl)butylamine

for 2-(5-methyl-4-imidazolylmethylthio)ethylamine in the procedure of Example 3(d) gives

11. 2-[4-(2-pyridyl)butylamino]-5-(2-hydroxy-4-pyridylmethyl)-4-pyrimidone
12. 2-[4-(3-chloro-2-pyridyl)butylamino]-5-(2-hydroxy-4-pyridylmethyl)-4-pyrimidone
13. 2-[4-(3-bromo-2-pyridyl)butylamino]-5-(2-hydroxy-4-pyridylmethyl)-4-pyrimidone.

Examples 14 to 17

Substitution of (i) 4-(2-pyridyl)butylamine
(ii) 4-(3-methoxy-2-pyridyl)butylamine
(iii) 4-(3-chloro-2-pyridyl)butylamine
(iv) 4-(3-bromo-2-pyridyl)butylamine

for 2-(5-methyl-4-imidazolylmethylthio)ethylamine

(a) in the procedure of Example 1(f)(g) gives

(i) 2-[4-(2-pyridyl)butylamino]-5-(6-hydroxy-3-pyridylmethyl)-4-pyrimidone
(ii) 2-[4-(3-methoxy-2-pyridyl)butylamino]-5-(6-hydroxy-3-pyridylmethyl)-4-pyrimidone
(iii) 2-[4-(3-chloro-2-pyridyl)butylamino]-5-(6-hydroxy-3-pyridylmethyl)-4-pyrimidone
(iv) 2-[4-(3-bromo-2-pyridyl)butylamino]-5-(6-hydroxy-3-pyridylmethyl)-4-pyrimidone

(b) in the procedure of Example 6(b)(c) gives

14. 2-[4-(2-pyridyl)butylamino]-5-(4-hydroxy-2-pyridylmethyl)-4-pyrimidone
15. 2-[4-(3-methoxy-2-pyridyl)butylamino]-5-(4-hydroxy-2-pyridylmethyl)-4-pyrimidone
16. 2-[4-(3-chloro-2-pyridyl)butylamino]-5-(4-hydroxy-2-pyridylmethyl)-4-pyrimidone
17. 2-[4-(3-bromo-2-pyridyl)butylamino]-5-(4-hydroxy-2-pyridylmethyl)-4-pyrimidone.

Examples 18 and 19

Substitution of 5,6 - dimethoxypyridine - 3 - carboxaldehyde for 6 - methoxypyridine - 3 - carboxaldehyde in the procedure of Example 1(b)-(e) gives 2-nitroamino-5-(5,6-dimethoxy-3-pyridylmethyl)-4-pyrimidone and this is reacted with 2-(5-methyl-4-imidazolylmethylthio)ethylamine or 4-(3-chloro-2-pyridyl)butylamine and the product heated under reflux in 2N hydrogen chloride in ethanol to give

18. 2-[2-(5-methyl-4-imidazolylmethylthio)ethylamino]-5-(6-hydroxy-5-methoxy-3-pyridylmethyl)-4-pyrimidone and
19. 2-[4-(3-chloro-2-pyridyl)butylamino]-5-(6-hydroxy-5-methoxy-3-pyridylmethyl)-4-pyrimidone.

*Preparation of pharmaceutical composition for oral administration*

A pharmaceutical composition is prepared containing

| | | % w/w |
|---|---|---|
| A | The product of any one of Example 1 to 19 | 55 |
| A | Dibasic calcium phosphate dihydrate | 20 |
| A | Approved colouring agent | 0.5 |
| | Polyvinylpyrrolidone | 4.0 |
| B | Microcrystalline cellulose | 8.0 |
| B | Maize starch glycollate | 8.0 |
| B | Sodium starch glycollate | 4.0 |
| B | Magnesium stearate | 0.5 |

by mixing tog...her the ingredients A (substituting lactose or microcrystalline cellulose for dibasic calcium phosphate dihydrate if desired), adding a concentrated solution of polyvinylpyrrolidone, and granulating, drying and screening the dried granules; adding the ingredients B to the dried granules and compressing the mixture into tablets, containing an amount of product corresponding to 100 mg, 150 mg or 200 mg of the free base.

*Preparation of pharmaceutical composition for topical administration*

A pharmaceutical composition is prepared containing

| | | % w/w |
|---|---|---|
| A | Stearyl alcohol | 15.0 |
| A | Beeswax | 8.0 |
| A | Sorbitan monooleate | 1.25 |
| A | Polyoxyethylene sorbitan monooleate | 3.75 |
| B | The product of any one of Examples 1 to 19 | 1.0 |
| B | Sorbitol solution B.P. | 7.5 |
| B | Citric acid | 0.2 |
| B | Sodium citrate | 0.05 |
| B | Methylparaben | 0.18 |
| B | Propylparaben | 0.02 |
| B | Water | to 100 |

A mixture of the ingedients A is heated to 72° and added with stirring to a mixture of the ingredients B at 70°, and the stirring is continued until a cream is formed.

## Claims

1. A pyrimidone of Structure (1):

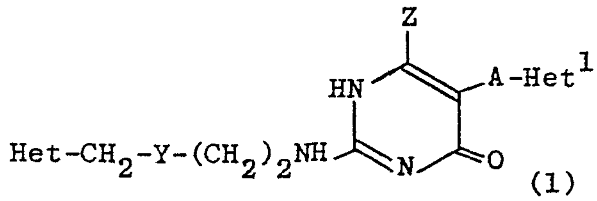

(1)

in which Het is a 2- or 4-imidazolyl group optionally substituted by $C_1$—$C_4$ alkyl, halogen, trifluoromethyl or hydroxymethyl, a 2-pyridyl group optionally substituted by one or more $C_1$—$C_4$ alkyl, $C_1$—$C_4$ alkoxy, halogen, amino or hydroxy groups, a 2-thiazolyl group, a 3-isothiazolyl group optionally substituted by chlorine or bromine, a 3-(1,2,5)-thiadiazolyl group optionally substituted by chlorine or bromine, or a 2-(5-amino-1,3,4-thiadiazolyl) group; Y is sulphur or methylene; Z is hydrogen or $C_1$—$C_4$ alkyl; A is $C_1$—$C_5$ alkylene; and $Het^1$ is a pyridyl group substituted by hydroxy or N-oxo and optionally substituted by $C_1$—$C_4$ alkyl or $C_1$—$C_4$ alkoxy, or a pharmaceutically acceptable acid addition salt thereof.

2. A compound according to Claim 1 in which Het is a 5-methyl-4-imidazolyl, 5-bromo-4-imidazolyl, 2-pyridyl, 3-methyl-2-pyridyl, 3-methoxy-2-pyridyl, 3-ethoxy-2-pyridyl, 3,4-dimethoxy-2-pyridyl, 3-fluoro-2-pyridyl, 3-chloro-2-pyridyl, 3-bromo-2-pyridyl, 3-iodo-2-pyridyl, 3-bromo-4-methyl-2-pyridyl or 2-thiazolyl group.

3. A compound according to Claim 1 or Claim 2 in which Z is hydrogen.

4. A compound according to any one of Claims 1 to 3 in which A is methylene.

5. A compound according to any one of Claims 1 to 4 in which $Het^1$ is 4-hydroxy-2-pyridyl, 6-hydroxy-3-pyridyl, 2-hydroxy-4-pyridyl, 4-hydroxy-5-methyl-2-pyridyl, 6-hydroxy-5-methyl-3-pyridyl, 2-hydroxy-6-methyl-4-pyridyl, 6-hydroxy-5-methoxy-3-pyridyl, N-oxo-3-pyridyl, N-oxo-6-methyl-3-pyridyl or N-oxo-4-pyridyl.

6. A compound according to Claim 1 in which Het is 5-methyl-4-imidazolyl, 3-methoxy-2-pyridyl, 3-bromo-2-pyridyl, or 2-thiazolyl, Z is hydrogen, A is methylene and $Het^1$ is 2-hydroxy-4-pyridyl.

7. 2 - [2 - (5 - Methyl - 4 - imidazolylmethylthio)ethylamino] - 5 - (2 - hydroxy - 4 - pyridylmethyl)-4 - pyrimidone, or a pharmaceutically acceptable acid addition salt thereof.

8. 2-[2-(2-Thiazolylmethylthio)ethylamino]-5-(2-hydroxy-4-pyridylmethyl)-4-pyrimidone, or a pharmaceutically acceptable addition salt thereof.

9. A process for preparing a compound according to any one of Claims 1 to 8 which comprises reacting a compound of Structure (3):—

$$Het-CH_2-Y-(CH_2)_2NH_2$$

(2)

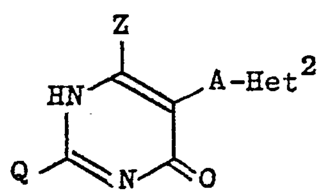

(3)

in which Q is nitroamino, $C_1$—$C_4$ alkylthio, benzylthio, chlorine, bromine or other group which can be displaced with a primary amine, and $Het^2$ is a pyridyl group substituted by hydroxy, a protected hydroxy group or N-oxo and optionally substituted by $C_1$—$C_4$ alkyl or $C_1$—$C_4$ alkoxy with an amine of Structure (2) and removal of any hydroxy protecting groups, and optionally converting the product into a pharmaceutically acceptable acid addition salt.

10. A pharmaceutical composition characterised in that it comprises a compound according to any one of Claims 1 to 8 and a pharmaceutically-acçeptable carrier or diluent.

**Revendications**

1. Pyrimidone de structure (1):

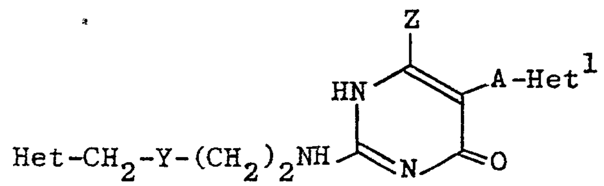

(1)

dans laquelle Het représente un groupe 2- ou 4-imidazolyle éventuellement substitué par un atome d'halogène, un groupe alcoyle en $C_1$—$C_4$, trifluorométhyle ou hydroxyméthyle, un groupe 2-pyridyle éventuellement substitué par un ou plusieurs groupes alcoyle en $C_1$—$C_4$, alcoxy en $C_1$—$C_4$, halogène, amino ou hydroxy, un groupe 2-thiazolyle, un groupe 3-isothiazolyle éventuellement substitué par un atome de chlore ou de brome, un groupe 3-(1,2,5)-thiadiazolyle éventuellement substitué par un atome de chlore ou de brome, ou un groupe 2-(5-amino-1,3,4-thiadiazolyl); Y représente un atome de soufre ou un radical méthylène; Z représente un atome d'hydrogène ou un groupe alcoyle en $C_1$—$C_4$; A représente un radical alocoylène en $C_1$—$C_5$; et Het$^1$ représente un groupe pyridyle substitué par un groupe hydroxy ou N- oxo et éventuellement substitué par un groupe alcoyle en $C_1$—$C_4$ ou alcoxy en $C_1$—$C_4$ ou un de ses sels d'addition d'acide pharmaceutiquement acceptable.

2. Composé suivant la revendication 1 dans laquelle Het représente un groupe 5-méthyl-4-imidazoyle, 5-bromo-4-imidazolyle, 2-pyridyle, 3-méthyl-2-pyridyle, 3-méthoxy-2-pyridyle, 3-éthoxy-2-pyridyle, 3,4-diméthoxy-2-pyridyle, 3-fluoro-2-pyridyle, 3-chloro-2-pyridyle, 3-bromo-2-pyridyle, 3-iodo-2-pyridyle, 3-bromo-4-méthyl-2-pyridyle ou 2-thiazolyle.

3. Composé suivant la revendication 1 ou la revendication 2 dans laquelle Z représente un atome d'hydrogène.

4. Composé suivant l'une quelconque des revendications 1 à 3 dans laquelle A représente un radical méthylène.

5. Composé suivant l'une quelconque des revendications 1 à 4 dans laquelle Het$^1$ représente un radical 4-hydroxy-2-pyridyle, 6-hydroxy-3-pyridyle, 2-hydroxy-4-pyridyle, 4-hydroxy-5-méthyl-2-pyridyle, 6-hydroxy-5-méthyl-3-pyridyle, 2-hydroxy-6-méthyl-4-pyridyle, 6-hydroxy-5-méthoxy-3-pyridyle, N- oxo -3-pyridyle, N- oxo -6-méthyl-3-pyridyle ou N- oxo -4-pyridyle.

6. Composé suivant la revendication 1 dans laquelle Het représente un groupe 5-méthyl-4-imidazolyle, 3-méthoxy-2-pyridyle, 3-bromo-2-pyridyle ou 2-thiazolyle, Z représente un atome d'hydrogène, A représente un radical méthylène et Het$^1$ représente un groupe 2-hydroxy-4-pyridyle.

7. 2-[2-(5-Méthyl-4-imidazolylméthylthio)éthylamino]-5-(2-hydroxy-4-pyridylméthyl)-4-pyrimidone ou un sel d'addition d'acide pharmaceutiquement acceptable de ce composé.

8. 2-[2-(2-Thiazolylméthylthio)éthylamino]-5-(2-hydroxy-4-pyridylméthyl)-4-pyrimidone ou un sel d'addition d'acide pharmaceutiquement acceptable de ce composé.

9. Procédé de préparation d'un composé suivant l'une quelconque des revendications 1 à 8 qui consiste à faire réagir un composé de structure (3)

$$Het\text{-}CH_2\text{-}Y\text{-}(CH_2)_2NH_2$$

(2)

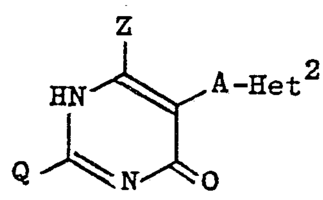

(3)

dans laquelle Q représente un atome de chlore, de brome, un groupe nitroamino, alcoyle en $C_1$—$C_4$ thio, benzylthio ou un autre groupe qui peut être déplacé par une amine primaire et Het$^2$ représente un groupe pyridyle substitué par un groupe hydroxy, un groupe hydroxy protégé ou N-oxo et éventuellement substitué par un groupe alcoyle en $C_1$—$C_4$ ou alcoxy en $C_1$—$C_4$, avec une amine de structure 2 et élimination de tous groupes hydroxy protecteurs et à transformer éventuellement le produit en un sel d'addition d'acide pharmaceutiquement acceptable.

10. Composition pharmaceutique caractérisée en ce qu'elle contient un composé suivant l'une quelconque des revendications 1 à 8 en association avec un excipient ou diluant pharmaceutiquement acceptable.

**Patentansprüche**

1. Pyrimidon der Strukturformel (I)

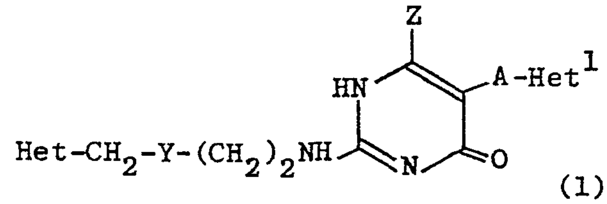

(1)

in der Het ein gegebenenfalls durch einen $C_{1-4}$-Alkylrest, ein Halogenatom oder eine Trifluormethyl- oder Hydroxymethylgruppe substituierte 2- oder 4-Imidazolylgruppe, eine gegebenenfalls durch einen $C_{1-4}$-Alkyl- oder $C_{1-4}$-Alkoxyrest, ein Halogenatom oder eine Amino- oder Hydroxygruppe ein- oder mehrfach substituierte 2-Pyridylgruppe, eine 2-Thiazolylgruppe, eine gegebenenfalls durch ein Chlor- oder Bromatom substituierte 3-Isothiazolylgruppe, eine gegebebenfalls durch ein Chlor- oder Bromatom substituierte 3-(1,2,5)-Thiadiazolylgruppe oder eine 2-(5-Amino-1,3,4-thiadiazolyl)-gruppe bedeutet; Y ein Schwefelatom oder eine Methylengruppe bedeutet; Z ein Wasserstoffatom oder einen $C_{1-4}$-Alkylrest bedeutet; A einen $C_{1-5}$-Alkylenrest bedeutet, und Het[1] eine durch eine Hydroxyl- oder N-Oxogruppe substituierte Pyridylgruppe darstellt, die gegebenenfalls durch einen $C_{1-4}$-Alkyl- oder $C_{1-4}$-Alkoxyrest substituiert ist, oder sein pharmazeutisch verträgliches Säureadditionssalz.

2. Verbindung nach Anspruch 1, in der Het eine 5-Methyl-4-imidazolyl-, 5-Brom-4-imidazolyl-, 2-Pyridyl-, 3-Methyl-2-pyridyl-, 3-Methoxy-2-pyridyl-, 3-Äthoxy-2-pyridyl-, 3,4-Dimethoxy-2-pyridyl-, 3-Fluor-2-pyridyl-, 3-Chlor-2-pyridyl-, 3-Brom-2-pyridyl-, 3-Jod-2-pyridyl-, 3-Brom-4-methyl-2-pyridyl- oder 2-Thiazolylgruppe bedeutet.

3. Verbindung nach Anspruch 1 oder 2, in der Z ein Wasserstoffatom bedeutet.

4. Verbindung nach einem der Ansprüche 1 bis 3, in der A eine Methylengruppe bedeutet.

5. Verbindung nach einem der Ansprüche 1 bis 4, in der Het[1] eine 4-Hydroxy-2-pyridyl-, 6-Hydroxy-3-pyridyl-, 2-Hydroxy-4-pyridyl-, 4-Hydroxy-5-methyl-2-pyridyl-, 6-Hydroxy-5-methyl-3-pyridyl-, 2-Hydroxy-6-methyl-4-pyridyl-, 6-Hydroxy-5-methoxy-3-pyridyl-, N-Oxo-3-pyridyl-, N-Oxo-6-methyl-3-pyridyl- oder N-Oxo-4-pyridylgruppe bedeutet.

6. Verbindung nach Anspruch 1, in der Het eine 5-Methyl-4-imidazolyl-, 3-Methoxy-2-pyridyl-, 3-Brom-2-pyridyl- oder 2-Thiazolylgruppe bedeutet, Z ein Wasserstoffatom bedeutet, A eine Methylengruppe bedeutet und Het[1] eine 2-Hydroxy-4-pyridylgruppe bedeutet.

7. 2-[2-(5-Methyl-4-imidazolylmethylthio)-äthylamino]-5-(2-hydroxy-4-pyridylmethyl)-4-pyrimidon oder sein pharmazeutisch verträgliches Säureadditionssalz.

8. 2-[2-(2-Thiazolylmethylthio)-äthylamino]-5-(2-hydroxy-4-pyridylmethyl)-4-pyrimidon oder sein pharmazeutisch verträgliches Säureadditionssalz.

9. Verfahren zur Herstellung einer Verbindung nach einem der Ansprüche 1 bis 8, gekennzeichnet durch Umsetzung einer Verbindung der Formel (III):

$$Het\text{-}CH_2\text{-}Y\text{-}(CH_2)_2NH_2$$

(2)

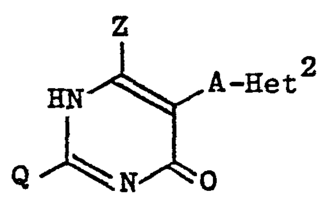

(3)

in der Q eine Nitroaminogruppe, einen $C_{1-4}$-Alkylthiorest, eine Benzylthiogruppe, ein Chlor- oder ein Bromatom oder andere Gruppen bedeutet, die durch ein primäres Amin ausgetauscht werden können, und Het[2] eine durch eine Hydroxylgruppe substituierte Pyridylgruppe, eine geschützte Hydroxylgruppe oder eine N-Oxogruppe bedeutet und gegebenenfalls durch einen $C_{1-4}$-Alkyl- oder $C_{1-4}$-Alkoxyrest substituiert ist, mit einem Amin der Formel (II) undanschließende Entfernung der Hydroxylschutzgruppen, und gegebenenfalls Überführung der erhaltenen Verbindung in ein pharmazeutisch verträgliches Säureadditionssalz.

10. Arzneimittel gekennzeichnet durch einen Gehalt einer Verbindung nach einem der Ansprüche 1 bis 8 und einem pharmazeutisch verträglichen Träger oder Verdünnungsmittel.